# EUROPEAN PATENT APPLICATION

(11) **EP 4 049 999 A1**
(43) Date of publication of application: **31.08.2022**
(21) Application number: 20879078.2
(22) Date of filing: 28.09.2020
(51) Int. Cl.: C07D 217/26, A61K 31/472, A61P 13/12, A61P 7/06

(54) **CRYSTAL FORM OF HYPOXIA-INDUCIBLE FACTOR-PROLYL HYDROXYLASE INHIBITOR**

(30) Priority: 22.10.2019 CN 201911007235; 17.01.2020 CN 202010057113; 19.03.2020 CN 202010198275
(71) Applicant: Crystal Pharmaceutical (Suzhou) Co., Ltd., Suzhou, Jiangsu 215123 (CN)
(72) Inventor: CHEN, Minhua, Suzhou, Jiangsu 215123 (CN); WU, Kelin, Suzhou, Jiangsu 215123 (CN); SHI, Jiaming, Suzhou, Jiangsu 215123 (CN)
(74) Representative: dompatent von Kreisler Selting Werner - Partnerschaft von Patent- und Rechtsanwälten mbB
(86) International application number: PCT/CN2020/118386
(87) International publication number: WO 2021/077994

(57) **Abstract**

A novel crystalline form of Compound I and preparation methods thereof, pharmaceutical compositions containing the crystalline form, and uses of the crystalline form for preparing hypoxia inducible factor prolyl hydroxylase inhibitor drugs and drugs for treating conditions mediated by hypoxia inducible factors. Compared with prior arts, the crystalline form of Compound I have one or more improved properties, which is of great value to the optimization and development of the drugs.

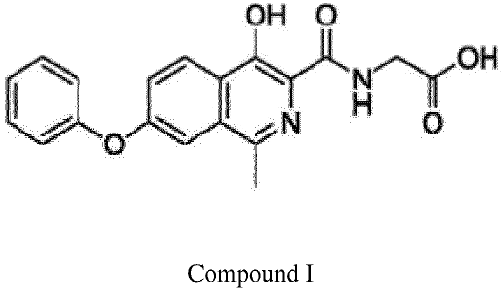

## Description

### TECHNICAL FIELD

The present disclosure pertains to the field of chemical crystallography, particularly relates to novel crystalline forms of Compound I, preparation method and use thereof.

### BACKGROUND

The cellular transcription factor HIF (Hypoxia Inducible Factor) occupies a central position in oxygen homeostasis in a wide range of organisms and is a key regulator of responses to hypoxia. The genes regulated by HIF transcriptional activity play critical roles in angiogenesis, erythropoiesis, hemoglobin F production, energy metabolism, inflammation, vasomotor function, apoptosis and cellular proliferation. HIF also plays a role in cancer (in tumor cells it is commonly upregulated), and in the pathophysiological responses to ischemia and hypoxia.

HIF prolyl hydroxylase inhibitors are useful for increasing the stability and/or activity of HIF, and useful for treating and preventing disorders associated with HIF, including anemia, ischemia, and hypoxia. The HIF prolyl hydroxylase inhibitor is developed by Fibrogen Inc., and was first approved in China in December 2018. It is marketed as a free form. Its chemical name is: [(4-hydroxy-1-methyl-7-phenoxy-isoquinoline-3-carbonyl)-amino]-acetic acid (Referred to as Compound I), and the structure is shown as follows:

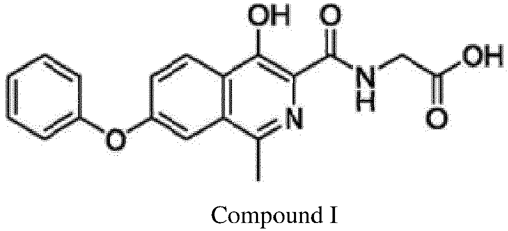

A crystalline form is a solid material whose constituents are arranged in a highly ordered microscopic structure. Polymorphism refers to the phenomenon that a compound exists in two or more than two crystalline forms. Different crystalline forms of drug substances have different physicochemical properties, which can affect drug's in vivo dissolution and absorption and will further affect drug's clinical efficacy and safety to some extent. In particular, for some poorly soluble solid, the above effects of the crystalline form will be greater. Therefore, polymorphism is an important part of drug research and drug quality control.

WO2014014835 disclosed amorphous, Form A, Form B, Form C and Form D of Compound I, wherein Form A is an anhydrate, Form B is a hemihydrate, Form C is a hexafluoropropan-2-ol solvate, and Form D is a DMSO: water solvate. Form C and Form D can hardly be used in drug products due to the solvent toxicity or excessive residue. Almost all Form B transforms into Form A after being stored at room temperature for one month. Form A is the most stable crystalline form among the four crystalline forms.

WO2019030711 disclosed several crystalline forms (Form δ, Form y) and co-crystals of Compound I, wherein Form γ is a formic acid and water mixed solvate. The content of formic acid in Form γ is 2-3% (w/w), which exceeds the ICH standard of solvent residue limit. Form δ is a hydrate containing 0.49% formic acid. Moreover, Form δ is unstable and will transform into WO2014014835 Form A when being heated.

CN109369525A disclosed twelve crystalline forms ARZ-A-ARZ-L of Compound I, wherein Form ARZ-A is the same as Form δ of WO2019030711. Form ARZ-B is the same as WO2014014835 Form A. Form ARZ-L is a crystalline form of Compound I hydrochloride. For the other crystalline forms disclosed in CN109369525A, the weight losses measured by TGA are all above 6%, indicating these crystalline forms have high content of solvent and are not suitable for industrial development. CN111320583A disclosed Form E/F/G/H and a DL-proline co-crystal of Compound I. Thereinto, Form E/F/G are the same as or mixtures of Form B of WO2014014835, which are unstable. Form H is the same as Form D of WO2014014835, which is a solvate. Form H is unstable and has solvent toxicity or solvent residue.

IN201641043301A disclosed Form α of Compound I. Form α is an acetic acid solvate with poor stability and great development difficulty, which is not conducive to industrialization. IN201841027602A disclosed three crystalline forms of Compound I, Form SR1, Form SR2 and Form SR3. The inventors of the present disclosure found that the preparation method disclosed in the patent has poor reproducibility by repeated experiments and is not conducive to industrialization. WO2019042641, WO2019042485, CN110218184A and IN201741007950A disclosed co-crystals of Compound I. The introduction of co-crystal formers other than the active ingredients in co-crystals increases the risk of drug side effects and is not conducive to industrialization.

IN201641016266A disclosed an amorphous solid dispersion of Compound I. The amorphous is in a thermodynamically unstable state, for the molecules in the amorphous are arranged in disorder. The amorphous is in a high-energy state and usually has poor stability. Amorphous drugs are prone to crystalline transformation during the production and storage process, which makes the bioavailability and dissolution rate of the drug lose consistency, leading to changes in the clinical efficacy of the drug. In addition, the preparation of amorphous is usually a process of rapid kinetic precipitation of solids, which easily leads to excessive residual solvents, and its particle properties are difficult to control through the process, making it highly challenging in the practical application of drugs.

WO2013013609 disclosed several crystalline forms of Compound I. It has been confirmed by FibroGen, Inc. that the compound in this patent is not Compound I, and does not disclose any crystalline form of Compound I.

Among all the crystalline forms of Compound I disclosed in the prior arts, WO2014014835 Form A has better properties compared with other crystalline forms. However, the inventors of the present disclosure found that the solubility of Form A is low, and the dissolution rate of Form A is slow, which is not conducive to the rapid and effective utilization of drugs. The crystallinity of Form A decreases after grinding.

From the above analysis of the prior arts, it can be seen that although there are many crystalline forms of Compound I, most of the crystalline forms have problems, such as poor stability, poor solubility, or unsuitability for industrial production, and so on. Therefore, it is necessary to further conduct polymorph screening of Compound I to find a crystalline form (preferably hydrate and anhydrate) that is more suitable for drug development.

In order to overcome the disadvantages of prior arts, the inventors of the present disclosure carried out a large number of experiments and surprisingly discovered the crystalline form CSI of Compound I. The crystalline form CSI is an anhydrate, and has advantages of physicochemical properties, formulation processing properties, and bioavailability, such as advantages in at least one aspect of melting point, solubility, hygroscopicity, purification ability, stability, adhesiveness, compressibility, flowability, in vivo and in vitro dissolution, bioavailability, etc. In particular, the crystalline form CSI of the Compound I of the present disclosure has advantages such as good solubility, dissolution rate, stability, hygroscopicity, compressibility and dissolution of the formulation, which provides a new and better choice for the development of drugs containing Compound I and is of great significance.

### Summary

The present disclosure is to provide novel crystalline forms of Compound I, preparation method and use.

According to the objective of the present disclosure, crystalline form CSI of Compound I is provided (hereinafter referred to as Form CSI).

In one aspect provided herein, the X-ray powder diffraction pattern of Form CSI comprises characteristic peaks at 2theta values of 5.4°±0.2°, 25.6°±0.2° and 27.4°±0.2° using CuKa radiation.

Furthermore, the X-ray powder diffraction pattern of Form CSI comprises one or two or three characteristic peaks at 2theta values of 10.8°±0.2°, 16.4°±0.2° and 24.0°±0.2° using CuKa radiation; preferably, the X-ray powder diffraction pattern of Form CSI comprises three characteristic peaks at 2theta values of 10.8°±0.2°, 16.4°±0.2° and 24.0°±0.2° using CuKa radiation.

Furthermore, the X-ray powder diffraction pattern of Form CSI comprises one or two or three characteristic peaks at 2theta values of 9.3°±0.2°, 11.7°±0.2° and 20.3°±0.2° using CuKa radiation; preferably, the X-ray powder diffraction pattern of Form CSI comprises three characteristic peaks at 2theta values of 9.3°±0.2°, 11.7°±0.2° and 20.3°±0.2° using CuKa radiation.

In another aspect provided herein, the X-ray powder diffraction pattern of Form CSI comprises three or four or five or six or seven or eight or nine or ten characteristic peaks at 2theta values at 5.4°±0.2°, 9.3°±0.2°, 10.8°±0.2°, 11.7°±0.2°, 16.4°±0.2°, 18.3°±0.2°, 20.3°±0.2°, 24.0°±0.2°, 25.6°±0.2° and 27.4°±0.2° using CuKa radiation.

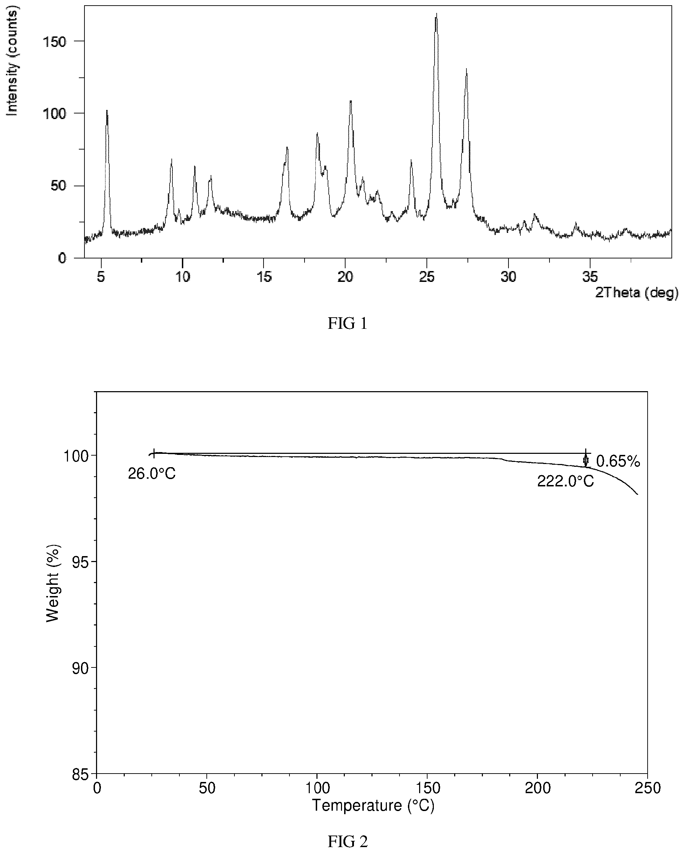

Without any limitation being implied, the XRPD pattern of Form CSI is substantially as depicted in Figure 1.

Without any limitation being implied, the TGA curve of Form CSI is substantially as depicted in Figure 2, which shows about 0.7% weight loss when heated to 222 °C.

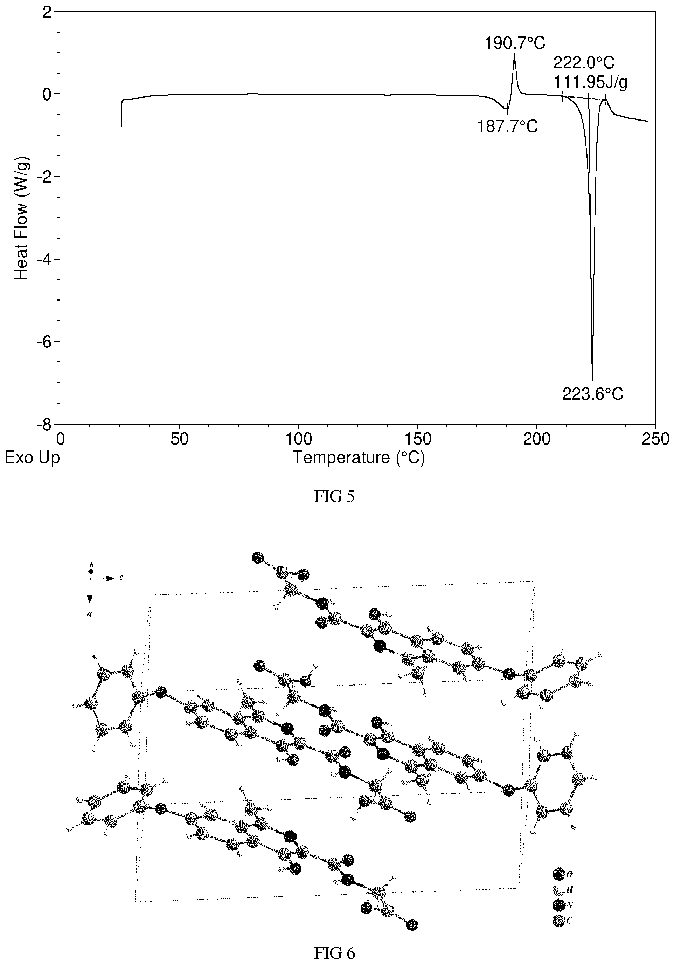

Without any limitation being implied, the DSC curve of Form CSI is substantially as depicted in Figure 5, which shows an endothermic peak at around 188 °C.

Without any limitation being implied, Form CSI is an anhydrate.

According to the objective of the present disclosure, a process for preparing Form CSI is also provided. The process comprises:
1) dissolving Compound I into an ester, filtering, and cooling the filtrate to obtain Form CSI; or
2) dissolving Compound I into an ester, or an acid, or a mixture of an ester and an acid, or a mixture of an acid and an ether, filtering, and evaporating the filtrate to obtain Form CSI; or
3) dissolving Compound I into an acid, placing the filtrate in an atmosphere of water vapor to obtain CSI by liquid vapor diffusion; or
4) dissolving Compound I into an acid, filtering, adding an alcohol into the filtrate slowly, standing or stirring to obtain Form CSI.

Furthermore, in method 1), said ester is preferably ethyl formate. Said dissolving temperature is preferably room temperature. Said cooling is preferably fast cooling.

Furthermore, in method 2), said ester is preferably ethyl formate. Said acid is preferably formic acid. Said ether is preferably methyl tert-butyl ether. Said dissolving temperature is preferably room temperature. The temperature of said evaporating is preferably room temperature.

Furthermore, in method 3), said acid is preferably formic acid. Said dissolving temperature is preferably 50-100 °C.

Furthermore, in method 4), said acid is preferably formic acid. Said alcohol is preferably ethanol. The temperature of said standing or stirring is preferably -20-40 °C, more preferably 5-30 °C.

Form CSI of the present disclosure has the following advantages:
1) Compared with prior arts, Form CSI has higher solubility. Particularly in SGF, the solubility of Form CSI is over six times that of WO2014014835 Form A.
   Higher solubility is beneficial to improve drug's in vivo absorption and bioavailability, thus improving drug efficacy. In addition, drug dose reduction without affecting efficacy is possible due to higher solubility, thereby reducing the drug's side effects and improving drug safety.
2) Compared with prior arts, Form CSI has a better dissolution rate and in vitro dissolution. In pH4.5 and pH6.8 PBS (Phosphate Buffered Saline), of Form CSI has a better intrinsic dissolution rate higher than that of WO2014014835 Form A. In pH4.5 ABS (Acetate Buffer Solution) and pH6.8 PBS, the dissolution of Form CSI drug product is higher than that of WO2014014835 Form A drug product.
   Drugs with different crystalline forms may lead to different in vivo dissolution, which directly affects the absorption, distribution, metabolism and excretion of the drug in vivo, and ultimately leads to different clinical efficacy due to their different bioavailability. Drug dissolution and dissolution rate are prerequisites for drug absorption. Good in vitro dissolution may lead to higher in vivo absorption, and better in vivo exposure, thereby improving drug's bioavailability and efficacy. Higher dissolution rate is beneficial for the drug to achieve peak concentration in plasma quickly after administration, thus ensuring rapid drug action.
3) Compared with prior arts, Form CSI of the present disclosure has lower hygroscopicity. The test results show that the weight gain of Form CSI at 80% RH (Relative humidity) is 0.12%, indicating that Form CSI is non hygroscopic or almost non-hygroscopic. The weight gain of WO2014014835 Form A at 80% RH is 0.21%, indicating that Form A is slightly hygroscopic.
   Hygroscopicity affects the physicochemical stability of the drug directly, as high hygroscopicity tends to cause chemical degradation and crystal transformation. In addition, high hygroscopicity will reduce the flowability of the drug, thereby affecting the processing of the drug. Moreover, drug substances with high hygroscopicity require low humidity environment during production and storage, which puts strict requirements on production and imposes higher costs. More importantly, high hygroscopicity is likely to cause variation in the content of active pharmaceutical ingredients in the drug, thus affecting drug quality. The crystalline form with low hygroscopicity is not demanding on the environment, which reduces the cost of production, storage and quality control, and has strong economic values.
4) Form CSI drug substance of the present disclosure has good stability itself and in drug product. Crystalline state of Form CSI drug substance doesn't change for at least three months when stored under the condition of 25 °C/60%RH. The chemical purity is above 99.5% and remains substantially unchanged during storage. After Form CSI is mixed with the excipients to form a drug product and stored under the condition of 25 °C/60%RH, crystalline state of Form CSI drug product doesn't change for at least three months. These results show that Form CSI drug substance has good stability under long term condition both itself and in drug product, which is beneficial to drug storage.
   Meanwhile, crystalline state of Form CSI drug substance doesn't change for at least three months when stored under the condition of 40 °C/75%RH. The crystalline state of Form CSI drug substance doesn't change for at least one month when stored under the condition of 60 °C/75%RH. The chemical purity is above 99.5% and remains substantially unchanged during storage. After Form CSI is mixed with the excipients to form a drug product and stored under the condition of 40 °C/75%RH, the crystalline state of Form CSI drug product doesn't change for at least three months. These results show that Form CSI drug substance has good stability under accelerated and stress conditions both itself and in drug product. Good stability under accelerated and stress conditions is of great importance to the drug development. Drug substance and drug product will go through high temperature and high humidity conditions caused by different seasons, regional climate and weather during storage, transportation and manufacturing processes. Form CSI drug substance and product have good stability under these stress conditions, which is beneficial to avoid the influence on drug quality when isn't stored in the conditions recommended in the label.
   Meanwhile, Form CSI has good mechanical stability. Form CSI has good physical stability after grinding. Grinding and pulverization are often required in the drug manufacturing process. Good physical stability of the drug substance can reduce the risk of crystallinity decrease and crystal transformation during the drug manufacturing process. Form CSI has good physical stability under different pressures, which is beneficial to keep crystalline form unchanged during tableting process. Crystalline form transformation can lead to changes in the absorption of the drug, affect bioavailability, and even cause toxicity and side effects. Good chemical stability ensures that no impurity would be generated during storage. Form CSI has good physical and chemical stability, ensuring consistent and controllable quality of the drug substance and drug product, and minimizing quality changes, bioavailability changes, toxicity and side effects caused by crystal transformation or impurity generation.

Furthermore, Form CSI of the present disclosure also has the following advantages:
Compared with prior arts, Form CSI of the present disclosure has better compressibility. Failure in hardness/friability test and tablet crack issue can be avoided due to better compressibility of Form CSI, making the preparation process more reliable, improving product appearance and product quality. Better compressibility can increase the compression rate, further increase the efficiency of process, and reduce the cost of compressibility improving excipients.

According to the objective of the present disclosure, a pharmaceutical composition is provided, said pharmaceutical composition comprises a therapeutically effective amount of Form CSI of Compound I and pharmaceutically acceptable carriers or excipients.

Furthermore, Form CSI of the present disclosure can be used for preparing hypoxia inducible factor prolyl hydroxylase inhibitor drugs.

Furthermore, Form CSI of the present disclosure can be used for preparing drugs treating a disease mediated by hypoxia inducible factor.

Furthermore, Form CSI of the present disclosure can be used for preparing drugs treating anemia caused by chronic kidney disease.

In the present disclosure, said "stirring" is accomplished by using a conventional method in the field such as magnetic stirring or mechanical stirring and the stirring speed is 50 to 1800 r/min. Preferably, the magnetic stirring speed is 300 to 900 r/min and mechanical stirring speed is 100 to 300 r/min.

Said "evaporating" is accomplished by using a conventional method in the field. For example, slow evaporation is accomplished in a container covered by a sealing film with pinholes. Rapid evaporation is accomplished in an open container.

Said "separation" is accomplished by using a conventional method in the field such as centrifugation or filtration. The operation of "centrifugation" is as follows: the sample to be separated is placed into a centrifuge tube, and then centrifuged at a rate of 10000 r/min until the solid all sink to the bottom of the tube.

Said "drying" is accomplished at room temperature or a higher temperature. The drying temperature is from room temperature to about 60° C, or to 50° C, or to 40° C. The drying time can be 2 to 48 hours, or overnight. Drying is accomplished in a fume hood, a forced air convection oven or a vacuum oven.

Said "rapid cooling" is accomplished by using a conventional method in the field. Rapid cooling is usually accomplished by transferring the sample directly from the environment not lower than room temperature to a refrigerator for cooling.

In the present disclosure, "crystal" or "crystalline form" refers to the solids being identified by the X-ray diffraction pattern. Those skilled in the art are able to understand that physicochemical properties discussed herein can be characterized. The experimental errors depend on the instrument conditions, the sample preparation and the purity of samples. In particular, those skilled in the art generally know that the X-ray diffraction pattern typically varies with the experimental conditions. It is necessary to point out that, the relative intensity of the diffraction peaks in the X-ray diffraction pattern may also vary with the experimental conditions. Therefore, the order of the diffraction peak intensities cannot be regarded as the sole or decisive factor. In fact, the relative intensity of the diffraction peaks in the X-ray powder diffraction pattern is related to the preferred orientation of the crystals, and the diffraction peak intensities shown herein are illustrative and identical diffraction peak intensities are not required. In addition, the experimental error of the diffraction peak position is usually 5% or less, and the error of these positions should also be considered. An error of ±0.2° is usually allowed. In addition, due to experimental factors such as sample thickness, the overall offset of the diffraction peak is caused, and a certain offset is usually allowed. Thus, it will be understood by those skilled in the art that a crystalline form of the present disclosure is not necessarily to have exactly the same X-ray diffraction pattern of the example shown herein. Any crystalline forms whose X-ray diffraction patterns have the same or similar characteristic peaks should be within the scope of the present disclosure. Those skilled in the art can compare the patterns shown in the present disclosure with that of an unknown crystalline form in order to identify whether these two groups of patterns reflect the same or different crystalline forms.

In some embodiments, Form CSI of the present disclosure is pure and substantially free of any other crystalline forms. In the present disclosure, the term "substantially free" when used to describe a novel crystalline form, it means that the content of other crystalline forms in the novel crystalline form is less than 20% (w/w), specifically less than 10% (w/w), more specifically less than 5% (w/w) and furthermore specifically less than 1% (w/w).

In the present disclosure, the term "about" when referring to a measurable value such as weight, time, temperature, and the like, is meant to encompass variations of ± 10%, ± 5%, ± 1%, ± 0.5%, or even ± 0.1% of the specified amount.

### BRIEF DESCRIPTION OF THE DRAWINGS

Figure 1 shows an XRPD pattern of Form CSI in example 1
Figure 2 shows a TGA curve of Form CSI in example 3
Figure 3 shows a ¹H NMR spectrum of Form CSI in example 3
Figure 4 shows an XRPD pattern of Form CSI in example 6
Figure 5 shows a DSC curve of Form CSI
Figure 6 shows a unit cell structure diagram of Form CSI
Figure 7 shows intrinsic dissolution profiles of Form CSI and WO2014014835 Form A in pH 6.8 PBS
Figure 8 shows intrinsic dissolution profiles of Form CSI and WO2014014835 Form A in pH 4.5 PBS
Figure 9 shows an XRPD pattern overlay of Form CSI before and after storage (from top to bottom: initial, stored at 25 °C/60%RH (sealed) for three months, stored at 25 °C/60%RH (open) for three months, stored at 40 °C/75%RH (sealed) for three months, stored at 40 °C/75%RH (open) for three months, stored at 60 °C/75%RH (sealed) for one month, stored at 60 °C/75%RH (open) for one month)
Figure 10 shows an XRPD pattern overlay of Form CSI before and after grinding (top: after grinding, bottom: before grinding)
Figure 11 shows an XRPD pattern overlay of WO2014014835 Form A before and after grinding (top: after grinding, bottom: before grinding)
Figure 12 shows an XRPD pattern overlay of Form CSI before and after DVS test (top: before DVS, bottom: after DVS).
Figure 13 shows an XRPD pattern overlay of Form CSI before and after formulation process (from top to bottom: excipients, Form CSI drug product, Form CSI).
Figure 14 shows dissolution curves of Form CSI drug product and WO2014014835 Form A drug product in pH4.5 ABS
Figure 15 shows dissolution curves of Form CSI drug product and WO2014014835 Form A drug product in pH6.8 PBS
Figure 16 shows an XRPD pattern overlay of Form CSI drug product (from top to bottom: stored in double aluminum blister under 40 °C/75%RH for three months, stored in double aluminum blister under 25 °C/60%RH for three months, initial drug product)
Figure 17 shows an XRPD pattern of Form K14 in example 20

### DETAILED DESCRIPTION

The present disclosure is further illustrated by the following examples which describe the preparation and use of the crystalline forms of the present disclosure in detail. It is obvious to those skilled in the art that changes in the materials and methods can be accomplished without departing from the scope of the present disclosure.

The abbreviations used in the present disclosure are explained as follows:
XRPD: X-ray Powder Diffraction
DSC: Differential Scanning Calorimetry
TGA: Thermo Gravimetric Analysis
DVS: Dynamic Vapor Sorption
¹H NMR: Proton Nuclear Magnetic Resonance
UPLC: Ultra Performance Liquid Chromatography
IDR: Intrinsic Dissolution Rate

Instruments and methods used for data collection:
X-ray powder diffraction patterns in the present disclosure were acquired by a Bruker D2 PHASER X-ray powder diffractometer. The parameters of the X-ray powder diffraction method are as follows:
X-Ray: Cu, Kα
Kα1 (Å): 1.54060; Kα2 (Å): 1.54439
Kα2/Kα1 intensity ratio: 0.50

Single crystal X-ray diffraction in the present disclosure was acquired by a BRUKER D8 QUEST X-ray diffractometer. The parameters of the single crystal X-ray diffraction are as follows:

| X-Ray Source | Microfocus Mo X-ray source (λ= 0.71073 Å) | |
|---|---|---|
| Detector | | CMOS Detector |
| Goniometer | | FIXED-CHI Goniometer |
| Cryogenic equipment | | Oxford Cryogenic System |
| Software package | | APEX3 |

Differential scanning calorimetry (DSC) data in the present disclosure were acquired by a TA Q2000. The parameters of the DSC method are as follows:
Heating rate: 10 °C/min
Purge gas: nitrogen

Thermo gravimetric analysis (TGA) data in the present disclosure were acquired by a TA Q500. The parameters of the TGA method are as follows:
Heating rate: 10 °C/min
Purge gas: nitrogen

Dynamic Vapor Sorption (DVS) was measured via an SMS (Surface Measurement Systems Ltd.) intrinsic DVS instrument. Its control software is DVS-Intrinsic control software. Typical parameters for DVS test are as follows:
Temperature: 25 °C
Gas and flow rate: N₂, 200 mL/min
dm/dt: 0.002%/min
RH range: 0% RH to 95% RH

Proton nuclear magnetic resonance spectrum data (¹H NMR) were collected from a Bruker Avance II DMX 400M HZ NMR spectrometer. 1-5 mg of sample was weighed and dissolved in 0.5 mL of deuterated dimethyl sulfoxide to obtain a solution with a concentration of 2-10 mg/mL.

The parameters for kinetic solubility tests of the present disclosure are as follows:

| | | | | |
|---|---|---|---|---|
| UPLC | Agilent 1290 with DAD detector | | | |
| Column | Waters ACQUITY UPLC BEH C₁₈ 2.1^{∗}50 mm, 1.7 µm | | | |
| Mobile phase | A : 0.1% trifluoroacetic acid aqueous solution | | | |
| | B : 0.1% trifluoroacetic acid acetonitrile solution | | | |
| Gradient | | Time (min) | | % A |
| | | 0.0 | | 80 |
| | | 0.3 | | 80 |
| | | 3.5 | | 40 |
| | | 8.0 | | 15 |
| | | 8.1 | | 80 |
| | | 10.0 | | 80 |
| Running time | 10.0 min | | | |
| Equilibrium time | 0.0 min | | | |
| Speed | 0.5 mL/min | | | |
| Injection volume | 1 µL | | | |
| Detection wavelength | UV at 224 nm | | | |
| Column temperature | 40 °C | | | |
| Sample temperature | Room temperature | | | |
| Diluent | Methanol | | | |

The parameters for purity tests of the present disclosure are as follows:

| | | | | |
|---|---|---|---|---|
| UPLC | Waters UPLC H-Class with DAD detector | | | |
| Column | ACE Excel 2 Super C₁₈ 3.0^{∗}100 mm, 2.0 µm | | | |
| Mobile phase | A: Acetonitrile: water (pH 3.0, H₃PO₄) = 5:95 | | | |
| | B: Acetonitrile | | | |
| Gradient | | Time (min) | | % A |
| | | 0.0 | | 40 |
| | | 0.5 | | 40 |
| | | 5.0 | | 20 |
| | | 7.0 | | 10 |
| | | 12.0 | | 10 |
| | | 12.1 | | 40 |
| | | 18.0 | | 40 |
| Running time | 18.0 min | | | |
| Equilibrium time | 0.0 min | | | |
| Speed | 0.5 mL/min | | | |
| Injection volume | 1 µL | | | |
| Detection wavelength | UV at 290 nm, 210 nm | | | |
| Column temperature | 40 °C | | | |
| Sample temperature | Room temperature | | | |
| Diluent | Methanol | | | |

The parameters for drug product dissolution tests of the present disclosure are as follows:

| | | |
|---|---|---|
| UPLC | Waters UPLC H-Class with DAD detector | |
| | Agilent 1290 with DAD detector | |
| Column | Waters ACQUITY UPLC BEH C₁₈ 2.1^{∗}50 mm, 1.7 µm | |
| Mobile phase | A : 0.1% trifluoroacetic acid aqueous solution | |
| | B : 0.1% trifluoroacetic acid acetonitrile solution | |
| Gradient | Time (min) | % A |
| | 0.0 | 80 |
| | 0.3 | 80 |
| | 3.5 | 40 |
| | 8.0 | 15 |
| | 8.1 | 80 |
| | 10.0 | 80 |
| Running time | 10.0 min | |
| Equilibrium time | 0.0 min | |
| Speed | 0.5 mL/min | |
| Injection volume | 5 µL | |
| Detection wavelength | UV at 224 nm | |
| Column temperature | 40 °C | |
| Sample temperature | Room temperature | |
| Diluent | Methanol | |

The parameters for intrinsic dissolution tests in the present disclosure are as follows:

| | | |
|---|---|---|
| UPLC | Agilent 1290 with DAD detector | |
| Column | Waters ACQUITY UPLC BEH C₁₈ 2.1^{∗}50 mm, 1.7 µm | |
| Mobile phase | A : 0.1% trifluoroacetic acid aqueous solution | |
| | B : 0.1% trifluoroacetic acid acetonitrile solution | |
| Gradient | Time (min) | % B |
| | 0.0 | 10 |
| | 0.3 | 10 |
| | 2.5 | 45 |
| | 6.0 | 80 |
| | 7.0 | 80 |
| | 7.1 | 10 |
| | 9.0 | 10 |
| Running time | 9.0 min | |
| Equilibrium time | 0.0 min | |
| Speed | 0.5 mL/min | |
| Injection volume | 20 µL | |
| Detection wavelength | UV at 250 nm | |
| Column temperature | 40 °C | |
| Sample temperature | Room temperature | |
| Diluent | pH4.5 PBS/pH6.8 PBS | |

Unless otherwise specified, the following examples were conducted at room temperature. Said "room temperature" is not a specific temperature, but a temperature range of 10-30° C.

According to the present disclosure, Compound I used as a raw material includes but not limited to solid (crystal or amorphous), oil, liquid and solution. Preferably, Compound I as a raw material is solid.

Compound I used in the following examples can be prepared by known methods in prior arts, for example, the method disclosed in WO2014014835.

### Examples

### Example 1 Preparation of Form CSI

5.3 mg of Compound I was weighed into a 5-mL glass vial, and 3 mL of ethyl formate was added thereto at room temperature. The solution was filtered through a 0.22 µm polytetrafluoron filter membrane after complete dissolution. The filtrate was cooled at 5 °C for about 89 hours, and then transferred to room temperature for fast evaporation for about 78 hours to obtain a solid. The solid is Form CSI. The XRPD pattern of Form CSI is substantially as depicted in Figure 1, and the XRPD data are listed in Table 1.

**Table 1**

| 2θ | d-spacing | Intensity % |
|---|---|---|
| 5.37 | 16.44 | 55.25 |
| 9.34 | 9.47 | 31.05 |
| 10.77 | 8.22 | 25.85 |
| 11.71 | 7.56 | 19.00 |
| 16.42 | 5.40 | 33.58 |
| 18.27 | 4.86 | 40.98 |
| 18.82 | 4.72 | 24.60 |
| 20.32 | 4.37 | 57.09 |
| 21.05 | 4.22 | 20.81 |
| 21.93 | 4.05 | 14.16 |
| 24.03 | 3.70 | 31.00 |
| 25.57 | 3.48 | 100.00 |
| 27.44 | 3.25 | 71.31 |
| 31.80 | 2.81 | 5.51 |

### Example 2 Preparation of Form CSI

135.1 mg of Compound I was weighed into a 100-mL glass vial, and completely dissolved in 60 mL of ethyl formate/formic acid (4:1, v/v) at room temperature. The filtrate was filtered through a 0.22 µm filter membrane and the filtrate was placed in a petri dish with a diameter of 10 cm. Yellow solid was obtained by fast evaporation at room temperature. 21.0 mg of the yellow solid was weighed and placed in a 1.5-mL glass vial, and 0.5 mL of water was added thereto. The suspension was stirred at room temperature for 48 hours, followed by centrifugation. The solid was dried under vacuum at 50 °C for 5 hours. Form CSI was obtained.

The XRPD data of Form CSI are listed in Table 2.

**Table 2**

| 2θ | d-spacing | Intensity % |
|---|---|---|
| 5.37 | 16.44 | 96.61 |
| 9.33 | 9.48 | 17.54 |
| 10.71 | 8.26 | 23.05 |
| 11.75 | 7.53 | 11.90 |
| 16.19 | 5.48 | 17.88 |
| 16.42 | 5.40 | 24.99 |
| 18.29 | 4.85 | 30.16 |
| 20.40 | 4.35 | 44.88 |
| 21.13 | 4.20 | 10.85 |
| 22.02 | 4.04 | 11.64 |
| 24.09 | 3.69 | 14.80 |
| 25.61 | 3.48 | 100.00 |
| 27.48 | 3.25 | 60.85 |
| 31.68 | 2.82 | 4.52 |

### Example 3 Preparation of Form CSI

As the weight shown in Table 3, Compound I was weighed into glass vials and completely dissolved in the corresponding volume of ethyl formate/formic acid (4:1, v/v) at room temperature. The solutions were filtered by 0.22 µm filter membranes and then placed in petri dishes for fast evaporation at room temperature for a period of time to obtain yellow solids. The obtained solids were collected and put in a 5-mL glass vial, dried in vacuum at 50 °C for about 2 h, heated to 140 °C at 10 °C/min with TGA, and purged with nitrogen at 140 °C for 15 h to remove the residual solvent. Form CSI was obtained.

The XRPD data of Form CSI are listed in Table 4.

The TGA curve of Form CSI shows about 0.7% weight loss when heated to 222 °C, which is substantially as depicted in Figure 2.

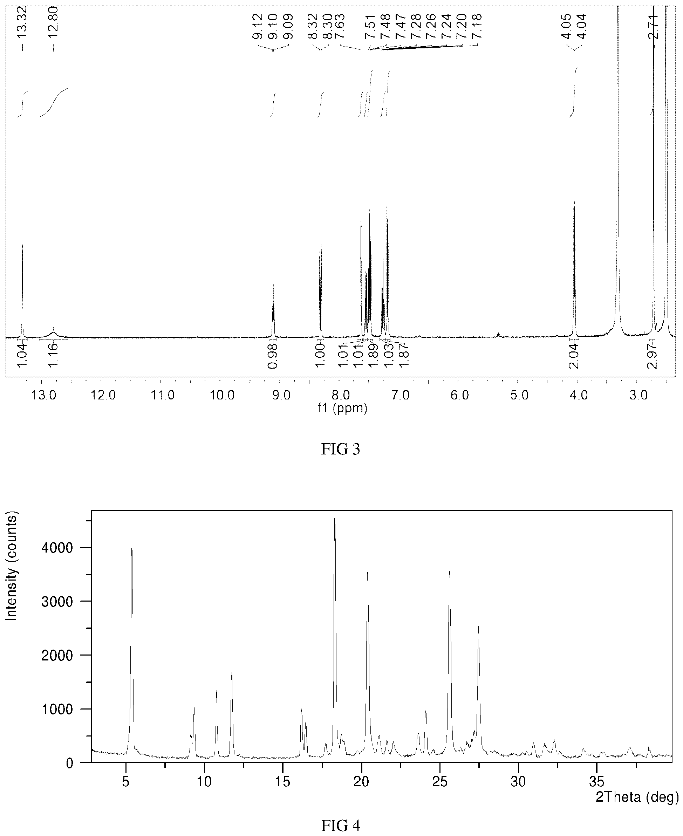

The ¹H NMR spectrum of Form CSI is substantially as depicted in Figure 3, the result shown is consistent with the structure of Compound I. The corresponding data are: ¹H NMR (400 MHz, DMSO) δ 13.32 (s, 1H), 12.80 (br, 1H), 9.10 (t, *J* = 6.0 Hz, 1H), 8.31 (d, *J* = 9.0 Hz, 1H), 7.63 (d, *J* = 2.3 Hz, 1H), 7.55 (dd, *J* = 9.0, 2.4 Hz, 1H), 7.52-7.46 (m, 2H), 7.26 (t, *J* = 7.4 Hz, 1H), 7.22-7.17 (m, 2H), 4.04 (d, *J* = 6.2 Hz, 2H), 2.71 (s, 3H).

**Table 3**

| Number | Weight (mg) | Solvent (v/v) | Volume (mL) | Diameter of petri dish (cm) | Evaporation time |
|---|---|---|---|---|---|
| 1 | 135.1 | Ethyl formate/Formic acid 4:1 | 60 | 10 | 20 hours |
| 2 | 1016.3 | Ethyl formate/Formic acid 4:1 | 500 | 20 | About 5 days |
| 3 | 135.9 | Ethyl formate/Formic acid 4:1 | 60 | 10 | About 3 days |
| 4 | 135.3 | Ethyl formate/Formic acid 4:1 | 60 | 10 | About 4 days |
| 5 | 134.5 | Ethyl formate/Formic acid 4:1 | 60 | 10 | About 4 days |
| 6 | 132.3 | Ethyl formate/Formic acid 4:1 | 60 | 10 | About 4 days |
| 7 | 136.8 | Ethyl formate/Formic acid 4:1 | 60 | 10 | About 4 days |
| 8 | 124.8 | Ethyl formate/Formic acid 4:1 | 60 | 10 | About 17 hours |
| 9 | 126.1 | Ethyl formate/Formic acid 4:1 | 60 | 10 | About 17 hours |
| 10 | 122.7 | Ethyl formate/Formic acid 4:1 | 60 | 10 | About 17 hours |

**Table 4**

| 2θ | d-spacing | Intensity % |
|---|---|---|
| 5.37 | 16.44 | 100.00 |
| 9.29 | 9.52 | 18.00 |
| 10.75 | 8.23 | 25.64 |
| 11.72 | 7.55 | 10.46 |
| 16.18 | 5.48 | 19.54 |
| 16.37 | 5.41 | 23.89 |
| 18.27 | 4.86 | 21.76 |
| 18.73 | 4.74 | 8.57 |
| 20.36 | 4.36 | 35.35 |
| 21.06 | 4.22 | 11.18 |
| 22.07 | 4.03 | 7.96 |
| 24.02 | 3.71 | 17.39 |
| 25.60 | 3.48 | 72.49 |
| 27.47 | 3.25 | 57.62 |
| 30.92 | 2.89 | 4.74 |
| 31.55 | 2.84 | 10.76 |
| 34.14 | 2.63 | 2.25 |
| 37.00 | 2.43 | 2.84 |
| 38.32 | 2.35 | 2.72 |

### Example 4 Preparation of Form CSI

74.4 mg of Compound I was weighed into a 3-mL glass vial, and completely dissolved in 1 mL of formic acid at 80 °C. Then the solution was cooled to room temperature and filtered into a glass tube through a 0.45 µm filter membrane.The glass tube was put into a glass vial containing 5 mL of water. The glass vial was capped and was placed at room temperature for about 4.5 days to obtain Form CSI.

The XRPD data of Form CSI are listed in Table 5.

**Table 5**

| 2θ | d-spacing | Intensity % |
|---|---|---|
| 5.37 | 16.44 | 25.79 |
| 9.44 | 9.37 | 23.13 |
| 10.77 | 8.21 | 11.50 |
| 11.79 | 7.50 | 18.97 |
| 16.18 | 5.48 | 10.07 |
| 16.50 | 5.37 | 12.27 |
| 17.83 | 4.98 | 8.13 |
| 18.30 | 4.85 | 100.00 |
| 18.51 | 4.79 | 46.58 |
| 18.89 | 4.70 | 14.80 |
| 20.48 | 4.34 | 71.28 |
| 21.23 | 4.19 | 13.90 |
| 22.07 | 4.03 | 6.84 |
| 23.66 | 3.76 | 8.74 |
| 24.12 | 3.69 | 15.08 |
| 24.67 | 3.61 | 4.57 |
| 25.60 | 3.48 | 65.33 |
| 27.46 | 3.25 | 44.41 |
| 30.64 | 2.92 | 4.51 |
| 31.02 | 2.88 | 5.52 |
| 31.82 | 2.81 | 6.89 |
| 32.44 | 2.76 | 6.16 |
| 34.20 | 2.62 | 6.14 |
| 35.46 | 2.53 | 2.56 |
| 37.09 | 2.42 | 5.70 |
| 38.39 | 2.34 | 2.51 |

### Example 5 Preparation of Form CSI

465.1 mg of Compound I was weighed into a 20-mL glass vial, and completely dissolved in 6.2 mL of formic acid at 80 °C. After filtration, 0.2 mL of filtrate was taken and placed in a glass vial. 0.2 mL of ethanol was slowly added into the glass vial at room temperature. Then the system was transferred to 5 °C and stirred overnight to obtain Form CSI.

The XRPD data of Form CSI are listed in Table 6.

**Table 6**

| 2θ | d-spacing | Intensity % |
|---|---|---|
| 5.37 | 16.44 | 100.00 |
| 9.13 | 9.69 | 13.14 |
| 9.32 | 9.49 | 63.51 |
| 10.77 | 8.21 | 30.56 |
| 11.73 | 7.55 | 23.41 |
| 12.18 | 7.26 | 4.18 |
| 16.20 | 5.47 | 18.36 |
| 16.42 | 5.40 | 41.56 |
| 17.69 | 5.01 | 10.88 |
| 18.30 | 4.85 | 88.95 |
| 18.70 | 4.75 | 20.12 |
| 20.39 | 4.36 | 50.64 |
| 21.12 | 4.21 | 17.74 |
| 21.64 | 4.11 | 6.25 |
| 23.58 | 3.77 | 4.08 |
| 24.07 | 3.70 | 2.87 |
| 24.53 | 3.63 | 6.60 |
| 25.60 | 3.48 | 18.61 |
| 26.30 | 3.39 | 5.24 |
| 26.70 | 3.34 | 4.35 |
| 27.47 | 3.25 | 11.17 |
| 28.22 | 3.16 | 3.21 |
| 30.48 | 2.93 | 2.93 |
| 31.57 | 2.83 | 5.03 |
| 32.29 | 2.77 | 2.88 |
| 37.10 | 2.42 | 3.32 |

### Example 6 Preparation of Form CSI

3.01 g of Compound I was dissolved in a mixed solvent comprised of 300 mL of formic acid and 1200 mL of methyl tert-butyl ether at room temperature. The solution was filtered through a medium-speed qualitative filter paper. The filtrate was fast evaporated at room temperature, and yellow solid was collected. The solid obtained is confirmed to be Form CSI. The XRPD pattern is substantially as depicted in Figure 4, and the XRPD data are listed in Table 7.

**Table 7**

| 2θ | d-spacing | Intensity % |
|---|---|---|
| 5.37 | 16.44 | 87.95 |
| 9.12 | 9.70 | 9.88 |
| 9.35 | 9.45 | 21.22 |
| 10.77 | 8.22 | 28.18 |
| 11.75 | 7.53 | 36.50 |
| 16.18 | 5.48 | 20.75 |
| 16.45 | 5.39 | 14.79 |
| 17.72 | 5.00 | 5.84 |
| 18.29 | 4.85 | 100.00 |
| 18.73 | 4.74 | 9.37 |
| 20.40 | 4.35 | 77.48 |
| 21.13 | 4.20 | 8.79 |
| 21.63 | 4.11 | 6.81 |
| 22.04 | 4.03 | 6.05 |
| 23.64 | 3.76 | 9.55 |
| 24.10 | 3.69 | 19.35 |
| 25.61 | 3.48 | 76.78 |
| 26.73 | 3.33 | 5.24 |
| 27.48 | 3.25 | 53.99 |
| 30.95 | 2.89 | 4.86 |
| 31.63 | 2.83 | 4.84 |
| 32.28 | 2.77 | 6.68 |
| 34.13 | 2.63 | 3.65 |
| 35.38 | 2.54 | 1.65 |
| 37.07 | 2.43 | 3.84 |
| 38.33 | 2.35 | 3.65 |

### Example 7 Preparation of Form CSI

3.54 g of Compound I was dissolved in a mixed solvent comprised of 240 mL of formic acid and 80 mL of methyl tert-butyl ether at room temperature. The solution was filtered through a 0.45 µm polytetrafluoron filter membrane. The filtrate was fast evaporated at room temperature for 14.5 hours, and yellow solid was collected. The solid obtained is confirmed to be Form CSI.

### Example 8 DSC of Form CSI

About 2 mg of Form CSI was taken to test DSC. The DSC curve of Form CSI is substantially as depicted in Figure 5. An endothermic peak is observed at around 188 °C.

### Example 9 Single Crystal of Form CSI

1.4006 g of Compound I was weighed into a 20-mL glass vial, and 19.0 mL of formic acid was added to dissolve the solid. The solution of Compound I in formic acid was obtained after filtration. 1.0 mL of the solution obtained was put into a vial, and rod-like crystals were obtained after adding 1.0 mL of ethanol slowly into the system and standing at room temperature for 8 days. The rod-like solid obtained is confirmed to be Form CSI. Single crystal X-ray diffractometer was used for single crystal test and determination. The single crystal data of Form CSI are listed in Table 8, and the unit cell structure is shown in Figure 6. The results show that Form CSI is an anhydrate.

**Table 8**

| Crystallographic data and refinement parameters | |
|---|---|
| Empirical formula | C₁₉H₁₆N₂O₅ |
| Formula weight | 352.34 |
| Temperature | 193 K |
| Wavelength | Mο/Kα (λ = 0.71073 Å) |
| Crystal system, space group | Triclinic, P ,1 |
| Unit cell dimensions | a = 9.5448(7) Å |
| | b = 10.0048(7) Å |
| | c = 17.2755(12) Å |
| | a = 104.469(2)° |
| | β = 100.165(2)° |
| | γ = 93.031(2)° |
| Volume | 1564.15(19) Å³ |
| Z, calculated density | 4, 1.496 g/cm³ |
| Final R indices [I ≥ 2sigma(I)] | R1 = 0.0506, wR2 = 0.1428 |
| Final R indices [all data] | R1 = 0.0681, wR2 = 0.1525 |
| Largest diff. peak and hole | 0.74/-0.63 e.Å⁻³ |

### Example 10 Kinetic solubility of Form CSI

When solubility test is used to predict the in vivo performance of a drug, it is critical to simulate in vivo conditions as closely as possible. For oral medication, Simulated Gastric Fluid (SGF), Fasted-State Simulated Intestinal Fluid (FaSSIF), Fed-State Simulated Intestinal Fluid (FeSSIF) can be used to simulate the condition in vivo and predict the effects of feeding, thus solubility in such mediums is closer to that in vivo.

20 mg of Form CSI and 20 mg of WO2014014835 Form A were suspended into 4.0 mL of SGF, 4.0 mL of FaSSIF, 4.0 mL of FeSSIF and 4.0 mL of water to get saturated solutions. After equilibrated for 1 h, 4 h, 8 h and 24 h, the concentrations (µg/mL) of Compound I of the saturated solutions were measured by UPLC. The results are listed in Table 9.

**Table 9**

| Medium | Form A | | | | Form CSI | | | |
|---|---|---|---|---|---|---|---|---|
| | 1 hour µg/mL | 4 hours µg/mL | 8 hours µg/mL | 24 hours µg/mL | 1 hour µg/mL | 4 hours µg/mL | 8 hours µg/mL | 24 hours µg/mL |
| SGF | 3.6 | 3.7 | 3.7 | 3.6 | 22.7 | 24.0 | 24.2 | 24.0 |
| FeSSIF | 146.3 | 150.2 | 160.6 | 268.6 | 180.4 | 270.3 | 295.3 | 314.4 |
| FaSSIF | 241.2 | 569.1 | 567.3 | 570.5 | 617.0 | 934.6 | 1043.5 | 1109.6 |
| water | 4.7 | 7.9 | 8.4 | 10.1 | 18.2 | 21.9 | 34.1 | 25.7 |

The results show that the solubility of Form CSI in SGF, FaSSIF, FeSSIF and water is higher than that of Form A. Especially in SGF, the solubility of Form CSI is more than six times that of WO2014014835 Form A.

### Example 11 IDR of Form CSI

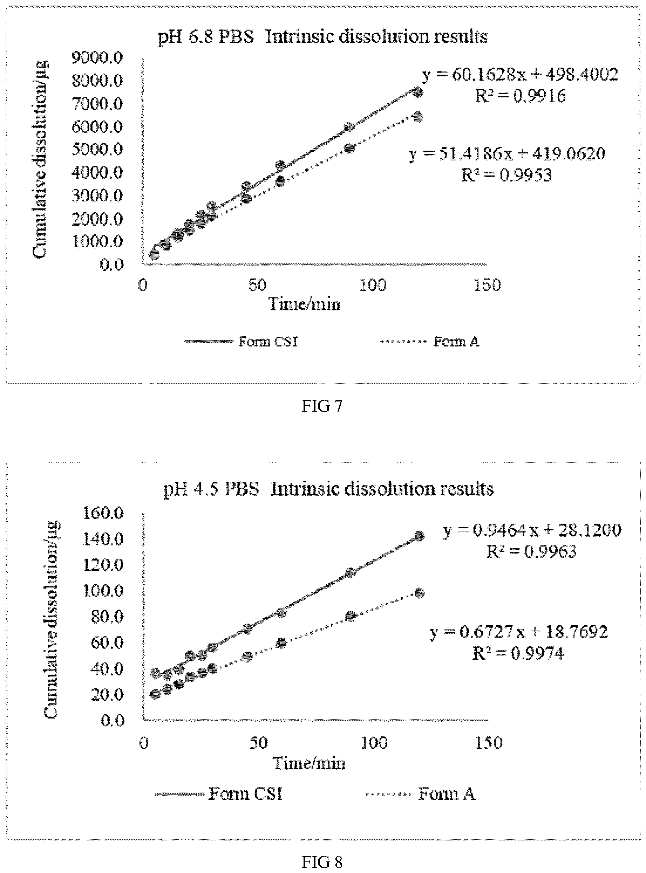

Approximately 100 mg of Form CSI or WO2014014835 Form A was added into the cavity of the die, and then compressed at 10 kN and held for 0.5 minute to obtain a tablet having a surface area of 0.5 cm². The die with the tablet still embedded was put into a dissolution apparatus to test the intrinsic dissolution. Dissolution method is shown in Table 10. Dissolution profiles are presented in Figure 7 and Figure 8. Dissolution data are presented in Table 11. The slope (in µg /min) of the regression line was calculated according to the data within 5-120 minutes. IDR (in µg/min/cm²) was further calculated according to the slope. IDR results are presented in Table 12.

**Table 10**

| Instrument | Agilent 708DS |
|---|---|
| Medium | pH6.8 PBS/pH4.5 PBS |
| Volume | 900 mL |
| Speed | 100 rpm |
| Temperature | 37 °C |
| Sampling Time | 5, 10, 15, 20, 25, 30, 45, 60, 90, 120 min |
| Supplement medium | No |

**Table 11**

| Time (min) | Cumulative dissolution (µg) pH6.8 PBS | | Cumulative dissolution (µg) pH4.5 PBS | |
|---|---|---|---|---|
| | Form CSI | WO2014014835 Form A | Form CSI | WO2014014835 Form A |
| 5 | 440.4 | 439.4 | 36.8 | 19.9 |
| 10 | 916.0 | 815.5 | 35.6 | 24.1 |
| 15 | 1345.9 | 1158.2 | 39.8 | 28.7 |
| 20 | 1758.2 | 1488.8 | 50.0 | 34.0 |
| 25 | 2152.9 | 1800.6 | 50.4 | 36.6 |
| 30 | 2521.4 | 2110.0 | 56.0 | 40.2 |
| 45 | 3392.2 | 2853.7 | 70.7 | 49.1 |
| 60 | 4309.7 | 3624.8 | 83.0 | 59.4 |
| 90 | 5967.2 | 5068.5 | 114.0 | 80.0 |
| 120 | 7448.5 | 6427.1 | 142.3 | 98.2 |

| | | | | |
|---|---|---|---|---|
| Note : Slope was calculated by data within 5-120 minutes. | | | | |

**Table 12**

| Form | IDR (µg/min/cm²) | |
|---|---|---|
| | pH6.8 PBS | pH4.5 PBS |
| WO2014014835 Form A | 102.8372 | 1.3454 |
| Form CSI | 120.3256 | 1.8928 |

The results show that the dissolution rate of Form CSI in pH 6.8 and pH 4.5 PBS is higher than that of WO2014014835 Form A. Especially in pH 4.5 PBS, the dissolution rate of Form CSI is increased by 41% compared with the Form A in the prior art.

### Example 12 Stability of Form CSI

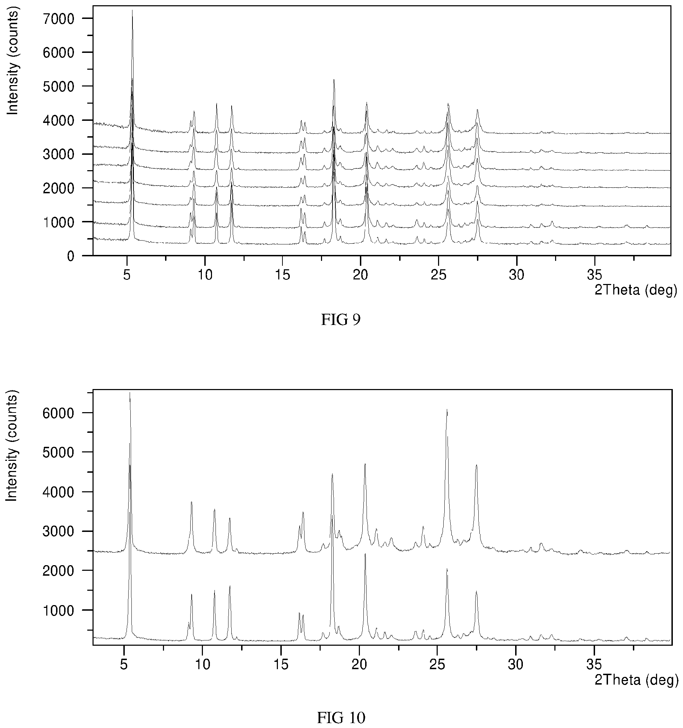

Approximately 5 mg of solid samples of Form CSI were stored under different conditions of 25 °C/60%RH, 40 °C/75%RH and 60 °C/75%RH. Chemical purity and crystalline form were checked by HPLC and XRPD, respectively. The results are shown in Table 13, and the XRPD overlay is shown in Figure 9.

**Table 13**

| Condition | | Time | Form | Purity |
|---|---|---|---|---|
| Initial | | - | Form CSI | 99.71% |
| 25 °C/60%RH | Sealed | 3 months | Form CSI | 99.65% |
| | Open | | Form CSI | 99.68% |
| 40 °C/75%RH | Sealed | 3 months | Form CSI | 99.66% |
| | Open | | Form CSI | 99.68% |
| 60 °C/75%RH | Sealed | 1 month | Form CSI | 99.68% |
| | Open | | Form CSI | 99.63% |

Form CSI kept stable for at least three months at 25 °C/60%RH and 40 °C/75%RH. Form CSI has good stability under both long-term and accelerated conditions. Form CSI kept stable for at least one month at 60 °C/75%RH. Form CSI has good stability under more stress conditions.

### Example 13 Physical stability of Form CSI on grinding

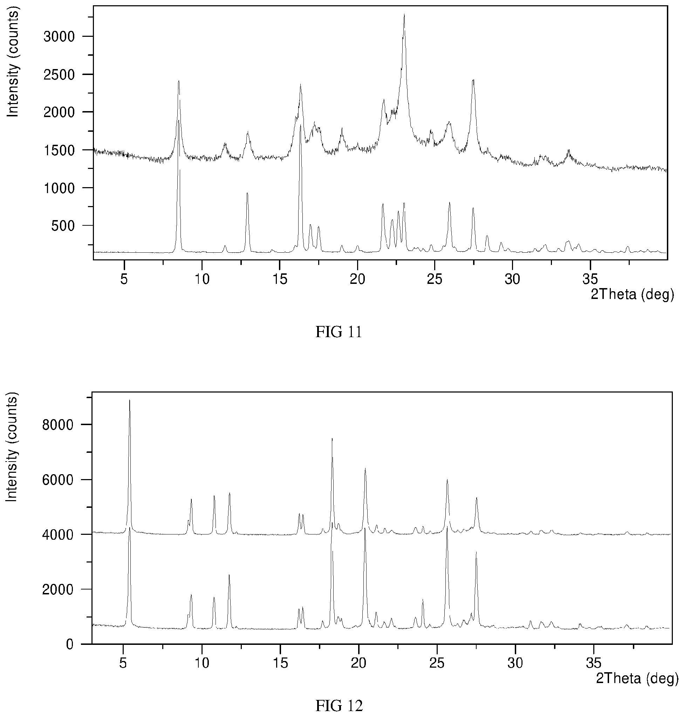

Form CSI and WO2014014835 Form A was ground manually for 5 min in a mortar. Crystalline forms were tested by XRPD before and after grinding, and the results are shown in Figure 10 and Figure 11.

The results show that the crystalline form and crystallinity of Form CSI of the present disclosure remain unchanged after grinding, while the crystallinity of WO2014014835 Form A decreases after grinding. Compared with WO2014014835 Form A, Form CSI shows better physical stability on grinding.

### Example 14 Pressure stability of Form CSI

Certain amount of Form CSI was compressed into tablets under different pressures with suitable tableting die. Crystalline forms before and after tableting were checked by XRPD. The test results are shown in Table 14.

**Table 14**

| Before tableting | Pressure | After tableting |
|---|---|---|
| Form CSI | 10 kN | Form CSI |
| | 15 kN | Form CSI |

The results show that Form CSI has good stability under different pressures.

### Example 15 Hygroscopicity of Form CSI

DVS was applied to test the hygroscopicity of Form CSI and WO2014014835 Form A with about 10 mg of samples. The weight gains at each relative humidity were recorded in a cycle of 0-95%-0 RH. XRPD test was applied before and after DVS. The results are shown in Table 15. The XRPD patterns before and after DVS test of Form CSI are shown in Figure 12.

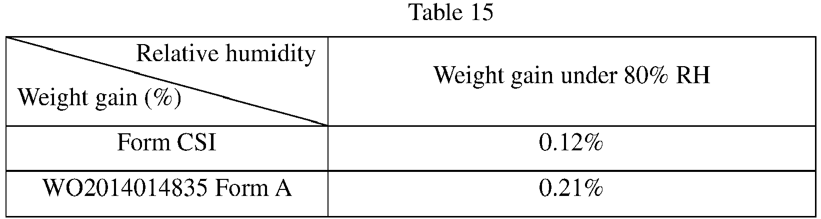

Description and definition of hygroscopicity (general notice 9103 drug hygroscopicity test guidelines in 2015 edition of Chinese Pharmacopoeia, test at 25 °C±1 °C, 80% RH. The definition of hygroscopicity in the 9th European Pharmacopoeia 5.11 is similar to the Chinese Pharmacopoeia.).
Deliquescent: sufficient water is absorbed to form a solution.
Very hygroscopic: increase in mass is equal to or greater than 15.0%.
Hygroscopic: increase in mass is less than 15.0% and equal to or greater than 2.0%.
Slightly hygroscopic: increase in mass is less than 2.0% and equal to or greater than 0.2%.
Non hygroscopic or almost non hygroscopic: increase in mass is less than 0.2%.

The results show that the weight gain of Form CSI under 80% RH is 0.12%. Form CSI is non-hygroscopic or almost non-hygroscopic. Weight gain of Form A in the prior art WO2014014835 under 80%RH is 0.21%. Form A is slightly hygroscopic. The hygroscopicity of Form CSI is superior to that of WO2014014835 Form A.

### Example 16 Compressibility of Form CSI

ENERPAC manual tablet press was used for compression. 80 mg of Form CSI and WO2014014835 Form A were weighed and added into the dies of a ϕ6 mm round tooling, compressed at 10 kN, then stored at room temperature for 24 h until complete elastic recovery. Hardness (H) was tested with an intelligent tablet hardness tester. Diameter (D) and thickness (L) were tested with a caliper. Tensile strength of the powder was calculated with the following formula: T=2H/πDL^{∗}9.8 Under a certain force, the greater the tensile strength, the better the compressibility. The results are presented in Table 16.

**Table 16**

| Form | Thickness (mm) | Diameter (mm) | Hardness (kgf) | Tensile strength (MPa) |
|---|---|---|---|---|
| WO2014014835 Form A | 2.21 | 5.78 | 0.67 | 0.33 |
| Form CSI | 2.10 | 6.01 | 2.47 | 1.22 |

The results indicate that Form CSI has better compressibility compared with WO2014014835 Form A.

### Example 17 Preparation of Form CSI drug product

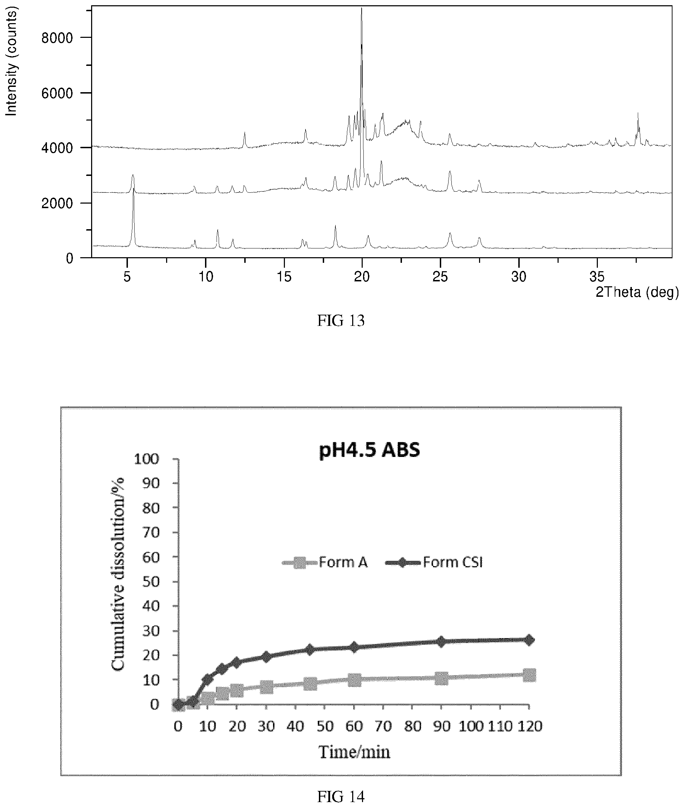

Form CSI of the present disclosure and WO2014014835 Form A were made into capsules using the formulation in Table 17 and the preparation process in Table 18. The XRPD patterns were collected before and after the formulation process. The XRPD overlay is shown in Figure 13. The results indicate that Form CSI remains stable before and after the formulation process.

**Table 17**

| No. | Component | mg/unit | % (w/w) | Function |
|---|---|---|---|---|
| 1 | Compound I | 20.00 | 22.22 | API |
| 2 | Microcrystalline Cellulose (Avicel PH 102) | 41.12 | 45.69 | Filler |
| 3 | Lactose monohydrate (Armor Pharma 150 mesh) | 20.33 | 22.59 | Filler |
| 4 | Povidone (PLASDONE K-29/32) | 2.70 | 3.00 | Binder |
| 5 | Croscarmellose sodium (Ac-Di-Sol SD-711) | 5.40 | 6.00 | Disintegrant |
| 6 | Glyceryl behenate (COMPRITOL 888ATO) | 0.45 | 0.50 | Lubricant |
| Total | | 90.00 | 100.00 | N/A |
| Note: There are two different crystalline forms of Compound I, Form CSI and WO2014014835 Form A, and their formulations are the same. | | | | |

**Table 18**

| Stage | Procedure |
|---|---|
| Blending | According to the formulation, materials No.1-6 were weighed into a 20-mL glass vial and blended manually for 2 mins. |
| Sifting | The mixture was pass through a 35-mesh sieve and then put in a 20-mL glass vial and mixed for 1 min. |
| Simulation of dry granulation | The mixture obtained was pressed by a single punch manual tablet press (type: ENERPAC, die: ϕ 20 mm round, flake weight: 500.0 mg±100.0 mg, pressure: 5 ± 1 kN). |
| Pulverizing | The flakes were pulverized and sieved through a 20-mesh sieve. |
| Final blending | The particles were placed in a 20-mL glass vial and mixed for 1 min. |
| Filling | The final blend (90 ± 5 mg) was weighed and filled into 4^{#} gelatin capsule. |
| Package | Capsules were sealed in double aluminum blisters. |

### Example 18 Dissolution of Form CSI drug product

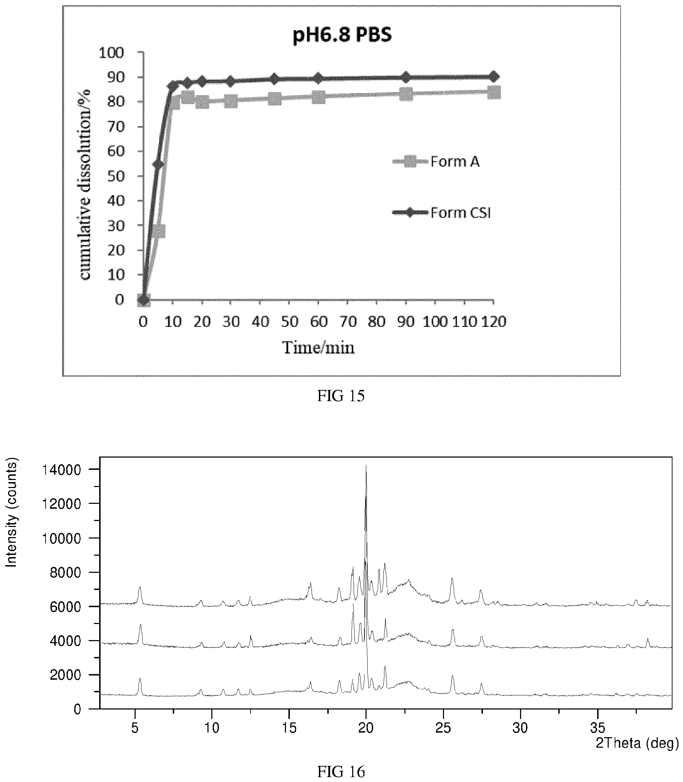

Dissolution tests were performed on Form CSI and WO2014014835 Form A drug products obtained from example 17. Dissolution method according to Chinese Pharmacopoeia 2015 0931Dissolution and release determination method was used. The conditions are listed in Table 19. The results are shown in Table 20, and the dissolution profiles are shown in Figure 14 and Figure 15.

**Table 19**

| Dissolution tester | Sotax AT7 |
|---|---|
| Method | Paddle |
| Strength | 20 mg |
| Medium | pH6.8 PBS/pH4.5 ABS |
| Volume | 900 mL |
| Speed | 50 rpm |
| Temperature | 37 °C |
| Time | 5, 10, 15, 20, 30, 45, 60, 90, 120 min |
| Supplementary medium | No |

**Table 20**

| Time (min) | pH4.5 ABS | | pH6.8 PBS | |
|---|---|---|---|---|
| | Cumulative drug release (%) | | | |
| | WO2014014835 Form A | Form CSI | WO2014014835 Form A | Form CSI |
| 0 | 0.0 | 0.0 | 0.0 | 0.0 |
| 5 | 0.9 | 1.4 | 27.9 | 54.9 |
| 10 | 2.7 | 10.1 | 79.8 | 86.3 |
| 15 | 4.6 | 14.3 | 81.9 | 87.7 |
| 20 | 5.9 | 17.0 | 80.1 | 88.3 |
| 30 | 7.3 | 19.4 | 80.6 | 88.4 |
| 45 | 8.6 | 22.2 | 81.3 | 89.3 |
| 60 | 10.1 | 23.2 | 82.0 | 89.5 |
| 90 | 10.8 | 25.5 | 83.2 | 90.0 |
| 120 | 12.2 | 26.4 | 84.0 | 90.2 |

Compound I is a free acid and belongs to a drug with pH-dependent solubility. The solubility in alkaline medium is relatively high, but the solubility in acidic medium is poor. Therefore, Form CSI and WO2014014835 Form A were not completely dissolved in pH 4.5 ABS, but the cumulative drug release of Form CSI is higher in pH4.5 ABS and pH6.8 PBS than that of WO2014014835 Form A. Therefore, it can be concluded that compared with WO2014014835 Form A, Form CSI of the present disclosure has better bioavailability.

### Example 19 Stability of Form CSI drug product

The Form CSI drug product was stored under 25 °C/60%RH and 40 °C/75%RH conditions for three months to evaluate its stability. The results are shown in Table 21. The XRPD pattern overlay before and after storage is shown in Figure 16.

**Table 21**

| Condition | Time | Form |
|---|---|---|
| Initial | - | Form CSI |
| 25 °C/60%RH, double aluminum blister | 3 months | Form CSI |
| 40 °C/75%RH, double aluminum blister | 3 months | Form CSI |

The results indicate that Form CSI drug product has good stability, for it can keep stable under 25 °C/60% RH and 40 °C/75% RH for at least three months.

### Example 20 Preparation of Type K14

362.5 mg of Compound I was weighed into a 10-mL glass vial, and 7.2 mL of hexafluoroisopropanol was added thereto at room temperature to dissolve the solid. A clear solution was obtained by filtration and then cooled to 5 °C with stirring. 3.6 mL of water was slowly added, and white solid was precipitated after stirring at 5 °C.

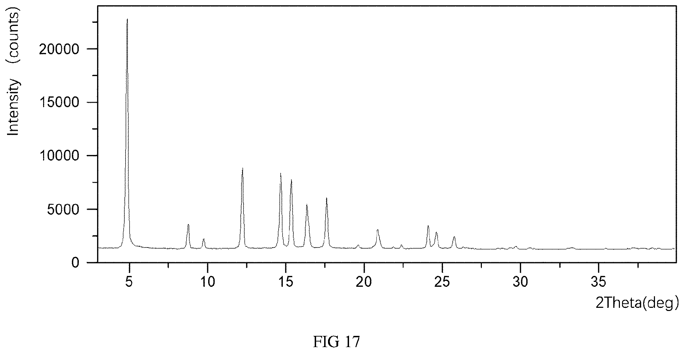

After test, the white solid obtained is confirmed to be a hexafluoroisopropanol solvate of Compound I. The XRPD pattern is substantially as depicted in Figure 17, and the XRPD data are listed in Table 22.

**Table 22**

| 2θ | d-spacing | Intensity % |
|---|---|---|
| 4.88 | 18.09 | 100.00 |
| 8.78 | 10.07 | 10.81 |
| 9.76 | 9.06 | 4.32 |
| 12.24 | 7.23 | 35.51 |
| 13.67 | 6.48 | 0.46 |
| 14.69 | 6.03 | 33.65 |
| 15.39 | 5.76 | 30.05 |
| 16.34 | 5.42 | 20.19 |
| 16.49 | 5.37 | 10.30 |
| 17.63 | 5.03 | 22.45 |
| 19.63 | 4.52 | 1.65 |
| 20.88 | 4.25 | 8.55 |
| 21.87 | 4.06 | 0.95 |
| 22.39 | 3.97 | 1.73 |
| 24.10 | 3.69 | 10.43 |
| 24.63 | 3.61 | 7.43 |
| 25.78 | 3.46 | 5.55 |
| 26.33 | 3.38 | 0.90 |
| 26.60 | 3.35 | 0.62 |
| 28.85 | 3.09 | 0.40 |
| 29.35 | 3.04 | 0.53 |
| 29.71 | 3.01 | 1.28 |
| 30.63 | 2.92 | 0.44 |
| 30.94 | 2.89 | 0.20 |
| 33.32 | 2.69 | 0.64 |
| 35.48 | 2.53 | 0.17 |
| 37.17 | 2.42 | 0.46 |
| 38.42 | 2.34 | 0.44 |

The examples described above are only for illustrating the technical concepts and features of the present disclosure and intended to make those skilled in the art being able to understand the present disclosure and thereby implement it and should not be concluded to limit the protective scope of this disclosure. Any equivalent variations or modifications according to the spirit of the present disclosure should be covered by the protective scope of the present disclosure.

## Claims

1. A crystalline form CSI of Compound I, wherein the X-ray powder diffraction pattern comprises characteristic peaks at 2theta values of 5.4°±0.2°N 25.6°±0.2° and 27.4°±0.2° using CuKa radiation

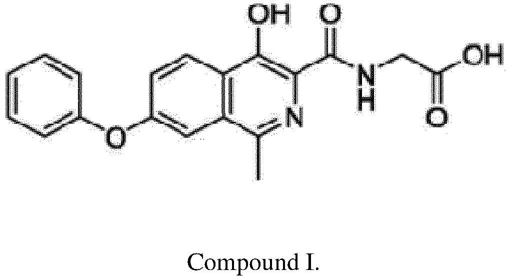

2. The crystalline form CSI according to claim 1, wherein the X-ray powder diffraction pattern comprises one or two or three characteristic peaks at 2theta values of 10.8°±0.2°, 16.4°±0.2° and 24.0°±0.2° using CuKa radiation.

3. The crystalline form CSI according to claim 1, wherein the X-ray powder diffraction pattern comprises one or two or three characteristic peaks at 2theta values of 9.3°±0.2°, 11.7°±0.2° and 20.3°±0.2° using CuKa radiation.

4. A process for preparing crystalline form CSI according to claim 1, wherein the process comprises:
1) dissolving Compound I into an ester, filtering, and cooling the filtrate to obtain crystalline form CSI; or
2) dissolving Compound I into an ester, or an acid, or a mixture of an ester and an acid, or a mixture of an acid and an ether, filtering, and evaporating the filtrate to obtain crystalline form CSI; or
3) dissolving Compound I into an acid, placing the filtrate in an atmosphere of water vapor to obtain crystalline form CSI by liquid vapor diffusion; or
4) dissolving Compound I into an acid, filtering, adding an alcohol into the filtrate slowly, standing or stirring to obtain crystalline form CSI.

5. The process according to claim 4, wherein said ester is ethyl formate, said acid is formic acid, said ether is methyl tert-butyl ether, said alcohol is ethanol.

6. A pharmaceutical composition, wherein said pharmaceutical composition comprises a therapeutically effective amount of crystalline form CSI according to claim 1, and pharmaceutically acceptable carriers or excipients.

7. The use of crystalline form CSI according to claim 1 for preparing hypoxia inducible factor prolyl hydroxylase inhibitor drugs.

8. The use of crystalline form CSI according to claim 1 for preparing drugs treating a disease mediated by hypoxia inducible factor.

9. The use of crystalline form CSI according to claim 1 for preparing drugs treating anemia caused by chronic kidney disease.
